# EUROPÄISCHE PATENTSCHRIFT

(11) **EP 0 472 767 B1**
(45) Veröffentlichungstag und Bekanntmachung des Hinweises auf die Patenterteilung: **15.03.1995**
(21) Anmeldenummer: 90116687.6
(22) Anmeldetag: 30.08.1990
(51) Int. Cl.: G01N 33/22, G01N 27/22

(54) **Vorrichtung zum Feststellen des Alkoholgehaltes oder des Heizwertes eines Gemischs**
Apparatus for the determination of the alcohol content or the calorific value of a mixture
Appareil pour la détermination du contenu en alcool ou la valeur calorifique d'un mélange

(43) Veröffentlichungstag der Anmeldung: 04.03.1992
(73) Patentinhaber: SIEMENS AKTIENGESELLSCHAFT, 80333 München (DE)
(72) Erfinder: Ertel, Gernot, Dipl.-Ing., D-8609 Bischberg (DE); Brabetz, Ludwig, Dr. rer. nat., D-8500 Nürnberg (DE); Schwarz, Waldemar, D-8501 Schwarzenbruck (DE)

(56) Entgegenhaltungen:
- EP-A- 0 380 751
- DE-A- 3 923 992
- US-A- 4 939 468
- PATENT ABSTRACTS OF JAPAN, Band 5, Nr. 175 (P-88)[847], 11. November 1981/

## Beschreibung

Die Erfindung betrifft eine Vorrichtung nach dem Oberbegriff von Anspruch 1.

Ökonomische und ökologische Bestrebungen und der Wunsch nach sicherer Kraftstoffversorgung haben eine umfassende Suche nach alternativen Kraftstoffen ausgelöst. Da die Erdölressourcen begrenzt sind, ist man auf der Suche nach anderen alternativen Energieformen. Als eine Alternative hat sich Alkohol erwiesen, der aus nicht fossilen Energieträgern hergestellt werden kann. Da herkömmliche Kraftstoffe, wie z.B. Benzin, die aus Erdöl hergestellt sind, weit verbreitet sind, soll bei Brennkraftmaschinen mit Einspritzmotoren die Möglichkeit bestehen, dem Benzin in beliebigen Anteilen Alkohol beizumischen. Um eine einwandfreie Arbeitsweise einer solchen Brennkraftmaschine zu erreichen, ist die Kenntnis des Alkoholgehaltes, d.h. des Volumenanteils Alkohol im Gemisch erforderlich. Damit eine dem Betriebszustand angepaßte Kraftstoffmenge in den Verbrennungsraum eingespritzt werden kann, muß laufend der Alkoholgehalt bestimmt werden.

Aus der Patentschrift US-A 4 939 468 ist ein Sensor bekannt, mit dem das Mischungsverhältnis von Benzin und Alkohol in einem Kraftstoff über eine Messung der Dielektrizitätskonstante des Gemisches bestimmt wird. Das Gehäuse des Sensors bildet dabei die äußere Elektrode eines Kondensators, ein Sensor, der durch isolierende und abdichtende Stützen im Gehäuse zentriert gehalten wird, bildet die innere Elektrode. Der Sensor ist mit einer außerhalb des Gehäuses liegenden Meßschaltung elektrisch verbunden.

Aus dem Dokument EP-A 0 380 752 ist eine Vorrichtung zum Messen des Alkoholgehaltes oder des Heizwertes von Kraftstoffen bekannt, die eine Meßzelle aufweist, in der ein von Kraftstoff benetzter Strömungskörper angeordnet ist. Der Strömungskörper hat auf seiner Außenseite eine erste Elektrode zum Messen der Dielektrizität des Kraftstoffes und eine zweite Elektrode zum Messen der Leitfähigkeit des Kraftstoffs. Das metallische Gehäuse der Meßzelle bildet die jeweilige Gegenelektrode. Eine mit den Elektroden verbundene Meßschaltung ist direkt an dem Gehäuse angebracht. Die Elektrode zur Kapazitätsmessung, ihr Außenanschluß und die Wand der Meßzelle bilden ein mechanisch starres System.

In der DE-OS 38 41 316 (entspricht EP-A 0 380 751) ist eine Vorrichtung zum Feststellen des Alkoholgehaltes und des Heizwertes eines Gemischs durch Messen von physikalischen Parametern des Gemischs in einem Gehäuse und mit einer elektronischen Meßschaltung beschrieben. Dabei stellen ein Mittelzylinder und ein Teil der Wandung eines Gehäuses die Elektroden eines Kondensators dar. Der Mittelzylinder wird über Zentrierscheiben vollständig im Gemisch gehalten. Im Inneren des Mittelzylinders befindet sich die Meßschaltung. Eine Spannungszuführung und eine Signalausgabe zu oder von der Meßschaltung verlaufen über die Zentrierscheiben.

Die als elektrische Verbindung von dem Mittelzylinder zu dem Gehäuse wirkenden Zentrierscheiben führen durch den vom Gemisch durchflossenen Raum. Da das Gemisch bei einem Einspritzmotor durch eine Kraftstoffpumpe unter hohem Druck gefördert wird, können durch pulsierendes Pumpen des Gemischs Schwingungen in axialer und radialer Richtung entstehen, die die Vorrichtung instabil werden lassen. Die über die Zentrierscheiben gehende Spannungszuführung und Signalausgabe sind daher auch anfällig gegen mechanische Einflüsse, wie z.B. Druckbeanspruchung. Des weiteren ist ein relativ hoher Fertigungsaufwand für das Zentrieren des Mittelzylinders im Gehäuse erforderlich.

Der Erfindung liegt die Aufgabe zugrunde, eine Vorrichtung zum Feststellen des Alkoholgehaltes oder des Heizwertes eines Gemischs gemäß dem Oberbegriff des Anspruches 1 zu schaffen, die vibrations- und druckfest ist.

Diese Aufgabe wird erfindungsgemäß durch eine Vorrichtung mit den Merkmalen des Patentanspruchs 1 gelöst.

Weitere Ausgestaltungen der Erfindung sind in den Unteransprüchen beschrieben.

Die Vorteile der Erfindung liegen unter anderem darin, daß die Meßschaltung gegen äußere Einflüsse, insbesondere elektromagnetischer, mechanischer und thermischer Art, geschützt ist und daß ihre Baugröße und die Fertigungskosten gering sind. Vorteilhaft wirkt sich außerdem das Einglasen eines Sensorelement in eine Zwischenplatte aus, da dadurch das Sensorelement vom Gehäuse isoliert wird und dieser Aufbau gleichzeitig vibrationsfest, druckfest, temperaturstabil und treibstoffresistent ist. Dies erspart sonstige, physikalisch und chemisch instabile Dichtungseinrichtungen. Das Einglasen des Sensorelements hat den weiteren Vorteil, daß durch die elektrisch leitende Wandung des Sensorelements eine elektrische Verbindung zu einer elektronischen Meßschaltung auf dem kürzesten Weg mit einer hohen mechanischen Festigkeit erreicht wird.

Ein Ausführungsbeispiel der Erfindung ist in den Zeichnungen dargestellt und wird im folgenden näher beschrieben. Es zeigen:
- Figur 1:: einen Querschnitt der erfindungsgemäßen Vorrichtung,
- Figur 2:: zwei Sensorelemente, die in einer Zwischenplatte der Vorrichtung nach Figur 1 eingeglast sind,
- Figur 3:: einen Querschnitt eines Gehäuses der Vorrichtung nach Figur 1,
- Figur 4:: die Untersicht des Gehäuses nach Figur 3,
- Figur 5:: die relative Dielektrizitätskonstante εᵣ des Gemischs in Abhängigkeit von dem Alkoholgehalt und der Temperatur T als Kennfeld dargestellt.

Um den Alkoholgehalt oder den Heizwert eines Gemischs, in dem Alkohol und Benzin in beliebigen Anteilen enthalten sind, zu messen, muß mindestens ein physikalischer Parameter des Gemischs in einer Vorrichtung gemessen werden. Durch Messen der Kapazität C und der daraus berechneten relativen Dielektrizitätskonstanten εᵣ kann der Alkoholgehalt, z.B. als Volumenanteil Alkohol in dem Gemisch, ermittelt werden.

Die Dielektrizitätskonstante εᵣ des Gemischs hängt von den Volumenanteilen und den einzelnen Dielektrizitätskonstanten von Alkohol und Benzin ab. Die relative Dielektrizitätskonstante von Benzin beträgt etwa 2,05 bis 2,15 und von Alkohol, hier z.B. von Methanol, etwa 32.

Die Dielektrizitätskonstante εᵣ wird bestimmt durch Messen der Kapazität C eines Kondensators, der durch ein erstes Sensorelement 1 und einen Teil der Wandung eines Gehäuses 2 gebildet wird und der mit dem Gemisch als Dielektrikum gefüllt ist, sowie durch eine Referenzkapazität C_{O}, gemäß:

εᵣ = C / C_{O}.

Diese Referenzkapazität C_{O} wird vorab bestimmt mit dem gleichen Kondensator, jedoch ohne Dielektrikum, d.h. im Vakuum. Sie ist als Konstante in einer elektronischen Meßschaltung 3 abgelegt, beträgt etwa 3 bis 5 pF und berücksichtigt auch vorhandene parasitäre Kapazitäten.

Um eine vollständige Kompensation sekundärer Effekte, wie z.B. die Frequenzabhängigkeit der Kapazität C und die Temperaturabhängigkeit der Dielektrizitätskonstanten εᵣ, beim Bestimmen des Alkoholgehaltes in einem Gemisch zu erreichen, müssen die folgenden drei physikalischen Parameter gemessen werden: die Kapazität C und der Leitwert G zwischen einem Sensorelement und einem Teil der Wandung des Gehäuses 2, der dem Sensorelement gegenüber liegt, sowie die Temperatur T des Gemischs.

Die Kapazität C wird zwischen dem ersten Sensorelement 1 und dem gegenüberliegenden Teil des Gehäuses 2, das von dem Gemisch durchflossen ist, gemessen. Die Messung der Kapazität C wird z.B. durch Wasseranteile im Gemisch oder andere Verschmutzungen (Quereinflüsse) verfälscht. Diese Quereinflüsse auf die Kapazität C durch Verunreinigungen des Gemischs können durch die Kenntnis des Leitwertes G z.B. rechnerisch oder durch Verwendung einer Tabelle innerhalb eines Mikrocontrollers korrigiert werden. Der Leitwert G wird zwischen einem zweiten, gleich ausgebildeten Sensorelement 4 und einem weiteren Teil der Wandung des Gehäuses 2, der dem Sensorelement gegenüberliegt, bestimmt.

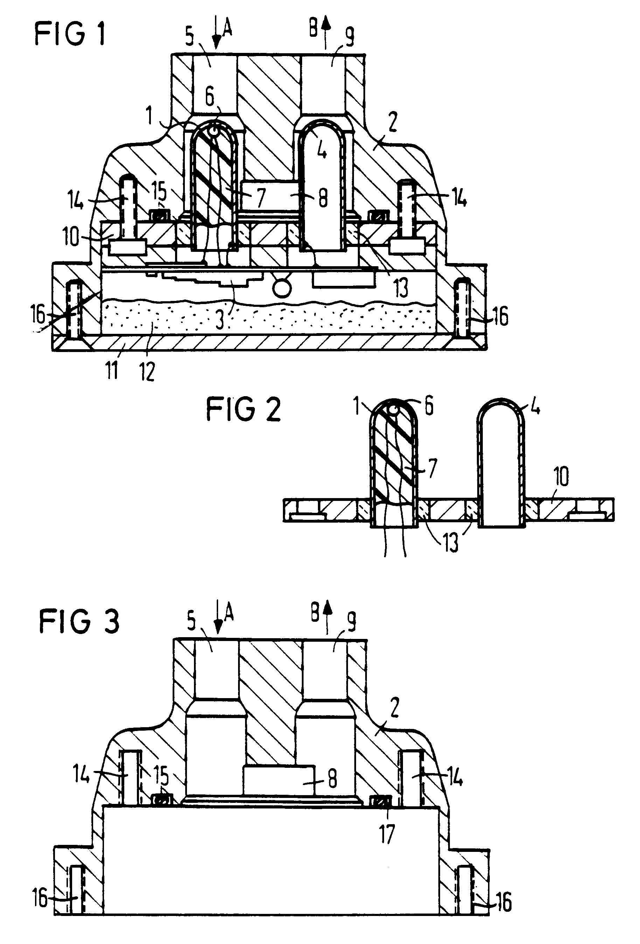

Das Gemisch strömt im Betrieb durch einen Kraftstoffzufluß 5, wobei die Strömungsrichtung durch die Pfeile A und B in Figur 1 gekennzeichnet ist, zu dem ersten Sensorelement 1, in dem ein Temperaturfühler 6 angebracht ist. Dieser Temperaturfühler 6, der als NTC-Widerstand ausgeführt ist, wird mit Wärmeleitpaste bestrichen und in die Spitze des ersten Sensorelements 1 gesteckt. Ein Kunststoffkörper 7 hält den Temperaturfühler 6 in seiner Lage. Die elektrischen Zuleitungen des Temperaturfühlers 6 führen auf direktem Weg zu der Meßschaltung 3.

Somit steht der Temperaturfühler 6 in thermischem Kontakt mit der Wandung des Sensorelements 1, deren Temperatur durch das vorbeiströmende Gemisch bestimmt wird. Bei vorgegebenem Mischungsverhältnis von Benzin und Alkohol wird vorab die Dielektrizitätskonstante εᵣ in Abhängigkeit von der Temperatur T gemessen und als Kennfeld in einem Speicherelement, z.B. einem ROM, der Meßschaltung 3 abgespeichert.

Über einen Kraftstoffkanal 8 gelangt das Gemisch vom ersten 1 zum zweiten Sensorelement 4 und schließlich zu einem Kraftstoffabfluß 9, der vor der Einspritzstelle im Motorraum liegt.

Das Gehäuse 2 ist so geformt, daß der Abstand zwischen einem der Sensorelemente 1 oder 4 und dem Gehäuse weitgehend gleich ist. Die in das Gemisch ragende Spitze eines der Sensorelemente 1 oder 4 ist so geformt, daß zum einen der Druckabfall kleiner als 0,1 bar ist bei einem Durchfluß von 3 l/min und daß zum anderen das Gemisch verwirbelt wird. Somit können Luftblasen, die durch die geringe Verflüchtigungstemperatur des Gemischs von etwa 80° C auf der Wandung eines der Sensorelemente 1 oder 4 oder des Gehäuses 2 entstehen, ausgeschwemmt werden, damit sie die Messung der Dielektrizitätskonstanten εᵣ nicht verfälschen.

Die beiden Sensorelemente 1 und 4 sind in einer Zwischenplatte 10 (siehe auch Figur 2) eingeglast, die den Raum, der mit dem Gemisch gefüllt ist, hermetisch zu der Meßschaltung 3 hin abdichtet. Die Einglasung 13 ist bis 70 bar druckfest.

Die Meßschaltung 3 wird über die Zwischenplatte 10 gekühlt, da die Temperatur des aus dem Kraftstofftank zufließenden Gemischs in der Regel kühler ist als die Temperatur im Motorraum.

Die Meßschaltung 3 befindet sich auf der Unterseite der Zwischenplatte 10 außerhalb des vom Gemisch durchflossenen Raums. Sie enthält einen Analogteil, in dem die physikalischen Parameter in entsprechende elektrische Signale gewandelt werden, und einen Digitalteil, in dem die gemessenen Daten durch ein Mikrocontroller ausgewertet werden und der berechnete Alkoholgehalt an eine Motorsteuerung weitergegeben wird.

Innerhalb eines Meßzyklus werden die Kapazität C und der Leitwert G der Meßanordnung mit dem Gemisch als Dielektrikum sowie die Temperatur T des Gemischs erfaßt und daraus der Alkoholgehalt festgestellt. Ein Meßzyklus dauert etwa 100 ms bei einer Taktfrequenz der Meßschaltung 3 von etwa 10 MHz. Der Alkoholgehalt wird der Motorsteuerung zur Steuerung der Einspritzmenge mitgeteilt entweder in Form einer Analogspannung, oder digital als moduliertes Signal.

Das Gehäuse 2 ist auf der Unterseite mit einer metallischen Bodenplatte 11 verschlossen. Dies stellt somit einen äußerlichen Schutz für die elektronische Meßschaltung 3 dar. Insbesondere wird dadurch die Meßschaltung 3 gegen elektromagnetische Felder abgeschirmt. Zusätzlich dichtet eine Schicht 12 aus Silikon das Gehäuse 2 ab und schützt die Meßschaltung 3 vor dem Oxidieren.

Die hülsenförmig ausgebildeten Sensorelemente 1, 4 (Figur 2) sind in die Zwischenplatte 10 eingeglast. Die Sensorelemente 1, 4 werden materialsparend als Tiefziehteile aus einer Nickel-Eisen-Legierung hergestellt; hier wird NiFe V540 verwendet. Dieses Material hat den Vorteil, daß eine Kontaktierung mit einem Draht zur Meßschaltung 3 hin gut möglich ist. Je ein Draht ist an das Sensorelement 1 und 4 angeschweißt und jeweils in der Meßschaltung 3 verlötet. Somit stellen die Sensorelemente 1 oder 4 sowohl eine Elektrode des Kondensators als auch eine elektrische Verbindung zwischen der Elektrode und der Meßschaltung 3 dar. Durch die geringe Wandstärke von etwa 1 mm findet ein guter Wärmeübergang von dem Gemisch über die Wandung des Sensorelements 1 zu dem Temperaturfühler 6 statt.

Die zylindrisch ausgebildeten Sensorelemente 1, 4 werden durch die Einglasung 13 zentrisch in einer Bohrung der Zwischenplatte 10 gehalten. Das Glas isoliert die Sensorelemente 1, 4 zur Zwischenplatte 10 hin. Glas ist nicht nur vibrationsfest und extrem druckfest, sondern auch resistent gegen das Gemisch und im spezifizierten Temperaturbereich von -40°C bis +125°C temperaturstabil. Durch das Einglasen sind die Sensorelemente 1, 4 von ihrer Lage her genau positioniert. Dies bringt eine Erleichterung bei der Montage der Vorrichtung.

Der Abstand zwischen dem Sensorelement 1 oder 4 und der Zwischenplatte 10 ist bei allen Vorrichtungen gleich. Dadurch ist die elektrische Reproduzierbarkeit gegeben. Der Kapazitätswert zwischen dem Sensorelement 1 und der Zwischenplatte 10 geht als parasitäre Kapazität in die Bestimmung der Dielektrizitätskonstanten εᵣ ein.

Die Zwischenplatte 10 ist genauso wie das Gehäuse 2 aus Aluminium im Druckguß hergestellt. Aluminium ist zwar leicht und kostengünstig, aber nicht resistent gegen das chemisch aggressive Gemisch. Daher wird es mit Kupfer und Nickel beschichtet. Es können ebenso auch andere Materialien verwendet werden, die elektrisch gut leitend und resistent gegen das Gemisch sind. Als Material für das Gehäuse kann auch ein Kunststoff verwendet werden, der mit einer Metallisierung, die als Elektrode wirkt, ausgestattet ist.

Figur 3 zeigt einen Querschnitt durch das Gehäuse 2 der Vorrichtung. Das Gemisch durchströmt das Gehäuse 2 in den mit den Pfeilen A, B angegebenen Richtungen. Ein Kraftstoffkanal 8 verbindet den Kraftstoffzufluß 5 mit dem Kraftstoffabfluß 9. Der Kraftstoffzufluß 5 und der Kraftstoffabfluß 9 weisen jeweils ein hier nicht dargestelltes Gewinde auf, mit dessen Hilfe jeweils eine Kraftstoffleitung vom Kraftstofftank kommend bzw. zu einer Einspritzstelle gehend angeschraubt wird.

Im Gehäuse 2 sind Befestigungsbohrungen 14 mit Gewinden vorgesehen, um die Zwischenplatte 10 am Gehäuse zu befestigen. Um das Gehäuses 2 zu der Meßschaltung 3 hin zusätzlich abzudichten, wird zwischen dem Gehäuse und der Zwischenplatte 10 ein O-Ring 15 eingepaßt.

Im unteren Teil des Gehäuses 2 befinden sich weitere Befestigungsbohrungen 16, die mit Gewinde versehen sind. Damit wird die Bodenplatte 11 an das Gehäuse 2 befestigt und somit die gesamte Vorrichtung abgedichtet. Das Gehäuse 2 ist elektrisch gut leitend und schirmt elektromagnetische Felder ab. Es ist ebenso feuchtigkeitsgeschützt.

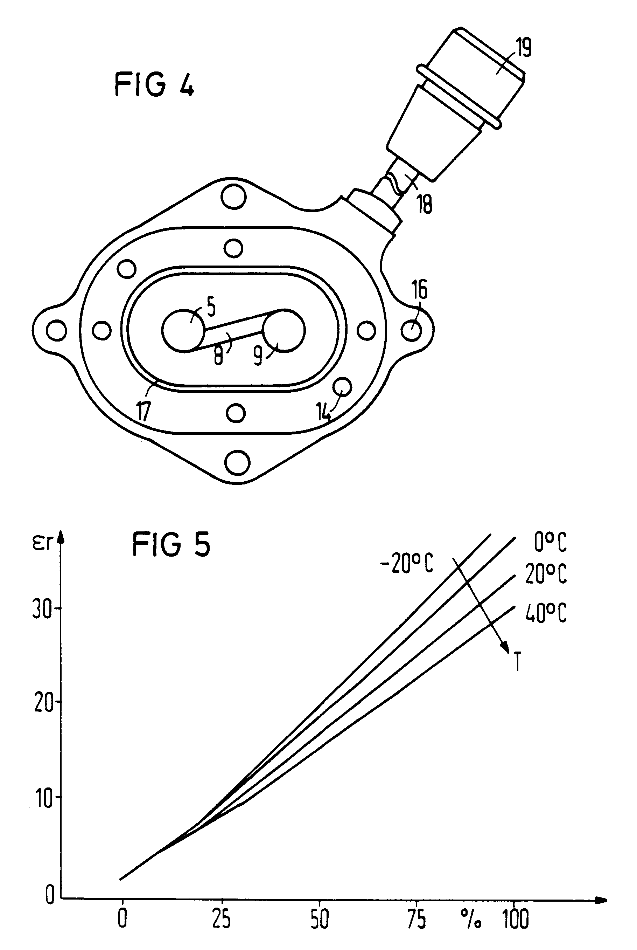

Der Kraftstoffkanal 8 (Figur 4) verläuft schräg zwischen dem Kraftstoffzufluß 5 und dem Kraftstoffabfluß 9. Dies versetzt das durchfließende Gemisch an dem zweiten Sensorelement 4 in Rotation. Damit werden schon bei moderaten Strömungen Luftblasen, die sich auf der Wandung des zweiten Sensorelements 4 oder auf der Wandung des Gehäuses 2 bilden können, ausgeschwemmt.

Der O-Ring 15 wird bei der Montage der Vorrichtung als Dichtungsring in eine Nut 17 eingelegt. Die Zwischenplatte 10 wird dann auf den inneren Teil des Gehäuses 2 aufgeschraubt und die Bodenplatte 11 auf den äußeren Teil. Die Befestigungsbohrungen 14, 16 hierzu sind in der Figur 4 zu sehen.

Am äußeren Rand des Gehäuses 2 befinden sich vier Flansche mit den Bohrungen 16, mittels derer die Bodenplatte 11 an dem Gehäuse 2 befestigt wird. Über ein Anschlußkabel 18 mit einem Stecker 19 wird die Vorrichtung elektrisch mit einer Motorsteuerung verbunden.

Das Kennfeld (Figur 5) zeigt die Abhängigkeit der relativen Dielektrizitätskonstanten εᵣ des Gemischs von dem Alkoholgehalt und der Temperatur T des Gemischs.

Zum Bestimmen des Alkoholgehaltes des Gemischs wird zuerst mit Hilfe des ersten Sensorelements 1 die Kapazität C zwischen dem Sensorelement und einem Teil der Wandung des Gehäuses 2 bestimmt. Parallel zu der Messung von C wird die Temperatur T des Gemischs mit dem Temperaturfühler 6 gemessen. Der Leitwert G wird mit Hilfe des zweiten Sensorelements 4 parallel dazu bestimmt. Danach wird die relative Dielektrizitätskonstante aus der gemessenen Kapazität C, die mit Hilfe des Leitwertes G korrigiert wird, und der gespeicherten Referenzkapazität C_{O} berechnet.

Aus der Dielektrizitätskonstanten εᵣ und der Temperatur T kann nun mit Hilfe des Kennfeldes der Alkoholgehalt ermittelt werden. Auf der x-Achse von Figur 5 ist der Alkoholgehalt in Prozent (%) des Gesamtvolumens des Gemischs aufgetragen und auf der y-Achse die Dielektrizitätskonstante εᵣ.

Mit dieser Vorrichtung besteht die Möglichkeit den Alkoholgehalt zwischen 0% und 100% Volumenanteilen Alkohol im Gemisch zu messen. Der Meßfehler bleibt dabei unter 5%.

Da Benzin und Alkohol einen unterschiedlichen Heizwert besitzen, kann über den Alkoholgehalt des Gemischs der Heizwert des Gemischs bestimmt werden. Der Heizwert von Benzin beträgt etwa 32 MJ/l und der von Methanol etwa 15,6 MJ/l.

Bei manchen Anwendungsfällen ist es günstiger, die erfindungsgemäße Vorrichtung zum Feststellen des Alkoholgehaltes nur mit einem Sensorelement zu versehen. Es werden dabei eine Kapazität C und ein Leitwert G zeitlich nacheinander gemessen.

In einem dritten Ausführungsbeispiel ist ein Kraftstoffzufluß und ein Kraftstoffabfluß horizontal ausgebildet. Das Gemisch strömt dann über die Spitze eines Sensorelements. Falls noch ein zweites Sensorelement vorhanden ist, strömt das Gemisch durch einen Kraftstoffkanal und dann über die Spitze des zweiten Sensorelements. Die Lage der Sensorelemente ist die gleiche wie im ersten Ausführungsbeispiel.

Bei allen drei Ausführungsbeispielen ist der Temperatursensor zur Messung der Temperatur T in demjenigen Sensorelement angeordnet, mit dem auch die Kapazität C gemessen wird.

## Patentansprüche

1. Vorrichtung zum Feststellen des Alkoholgehaltes oder des Heizwertes eines Gemischs, in dem Alkohol und Benzin enthalten sind, durch Messen von mindestens einem physikalischen Parameter des Gemischs in einem von dem Gemisch durchflossenen, elektrisch leitenden Gehäuse (2) und Auswerten der Messung in einer elektronischen Meßschaltung (3), die in dem Gehäuse (2) angeordnet ist, wobei ein Teil der Wandung des Gehäuses (2) eine erste Elektrode und ein elektrisch leitendes Sensorelement (1), das teilweise von dem Gemisch umgeben ist, eine zweite Elektrode eines Kondensators bildet,
**dadurch gekennzeichnet, daß**
- das Sensorelement (1), elektrisch mit der Meßschaltung (3) verbunden und in einer Zwischenplatte (10) hermetisch eingeglast ist, und
- das Gehäuse (2) durch die Zwischenplatte (10) zur Meßschaltung (3) hin hermetisch abgedichtet ist.

2. Vorrichtung nach Anspruch 1, dadurch gekennzeichnet, daß das Sensorelement (1) weithehend über seine gesamte Oberfläche den gleichen Abstand zu dem Gehäuse (2) aufweist.

3. Vorrichtung nach Anspruch 1, dadurch gekennzeichnet, daß das Sensorelement (1) hülsenförming ausgebildet ist und seine Wandstärke 1,5 mm oder weniger beträgt.

4. Vorrichtung nach Anspruch 1, dadurch gekennzeichnet, daß die meßschaltung (3) außerhalb des von dem Gemisch durchflossenen Raums auf der Zwischenplatte (10) angeordnet ist.

5. Vorrichtung nach Anspruch 1, dadurch gekennzeichnet, daß ein Temperaturfühler (6) in dem sensorelement (1) angeordnet ist und in Wärmekontakt mit der Wandung des Sensorelements (1) steht.

6. Vorrichtung nach Anspruch 1, dadurch gekennzeichnet, daß ein zweites Sensorelement (4) in der Zwischenplatte (10) befestigt ist.

7. Vorrichtung nach Anspruch 1, dadurch gekennzeichnet, daß das Gehäuse (2) zwischen dem ersten (1) und zweiten Sensorelement (4) einen Kraftstoffkanal (8) mit einer Schräge aufweist, die das durchfließende Gemisch in Rotation versetzt.

8. Vorrichtung nach Anspruch 1, dadurch gekennzeichnet, daß das Gehäuse (2) und die Zwischenplatte (10) aus Aluminium hergestellt und mit Kupfer und Nickel beschichtet sind.

9. Vorrichtung nach Anspruch 1, dadurch gekennzeichnet, daß das Sensorelement (1, 4) aus einer Eisen-Nickel-Legierung hergestellt ist.

## Claims

1. Device for determining the alcohol content or the calorific value of a mixture which contains alcohol and petrol, by measuring at least one physical parameter of the mixture in an electroconductive housing (2) through which the mixture flows, and evaluating the measurement in an electronic measuring circuit (3) which is arranged in the housing (2), a portion of the wall of the housing (2) forming a first electrode, and an electroconductive sensor element (1) which is partially surrounded by the mixture forming a second electrode of a capacitor, characterized in that
- the sensor element (1) is electrically connected to the measuring circuit (3) and is hermetically sealed, by means of a glass seal, in an intermediate plate (10), and
- the housing (2) is hermetically sealed by means of the intermediate plate (10) with respect to the measuring circuit (3).

2. Device according to Claim 1, characterized in that the sensor element (1) is at a substantially constant distance, over its entire surface, from the housing (2).

3. Device according to Claim 1, characterized in that the sensor element (1) is of a sleeve-type design and its wall thickness is 1.5 mm or less.

4. Device according to Claim 1, characterized in that the measuring circuit (3) is arranged on the intermediate plate (10) outside the space through which the mixture flows.

5. Device according to Claim 1, characterized in that a temperature sensor (6) is arranged in the sensor element (1) and is in thermal contact with the wall of the sensor element (1).

6. Device according to Claim 1, characterized in that a second sensor element (4) is fixed in the intermediate plate (10).

7. Device according to Claim 1, characterized in that the housing (2), between the first (1) and second sensor element (4), has a fuel duct (8) having a slope by which the mixture flowing through is set into rotation.

8. Device according to Claim 1, characterized in that the housing (2) and the intermediate plate (10) are made of aluminium and are coated with copper and nickel.

9. Device according to Claim 1, characterized in that the sensor element (1, 4) is made of an iron-nickel alloy.

## Revendications

1. Appareil de détermination de la teneur en alcool ou du pouvoir calorifique d'un mélange qui contient de l'alcool et de l'essence, par mesure d'au moins un paramètre physique du mélange dans un boîtier (2) conducteur de l'électricité et dans lequel passe le mélange et par exploitation de la mesure dans un circuit de mesure (3) électronique, qui est monté dans le boîtier (2), une partie de la paroi du boîtier (2) formant une première électrode et un élément (1) capteur, conducteur de l'électricité et entouré partiellement par le mélange, formant une seconde électrode d'un condensateur, caractérisé en ce que
- I'élément (1) capteur est relié électriquement au circuit de mesure (3) et est incorporé hermétiquement par vitrification dans une plaque (10) intermédiaire, et
- le boîtier (2) est rendu hermétiquement étanche vis-à-vis du circuit de mesure (3) par la plaque intermédiaire (10).

2. Appareil suivant la revendication 1, caractérisé en ce que sensiblement toute la surface de l'élément (1) détecteur est à la même distance du boîtier (2).

3. Appareil suivant la revendication 1, caractérisé en ce que l'élément (1) capteur est en forme de douille et son épaisseur de paroi est de 1,5 mm ou inférieure à 1,5 mm.

4. Appareil suivant la revendication 1, caractérisé en ce que le circuit de mesure (3) est monté sur la plaque intermédiaire (10) à l'extérieur de l'espace dans lequel passe le mélange.

5. Appareil suivant la revendication 1, caractérisé en ce qu'une sonde (6) de température est montée dans l'élément (1) capteur et est en contact thermique avec la paroi de l'élément (1) capteur.

6. Appareil suivant la revendication 1, caractérisé en ce qu'un deuxième élément (4) capteur est fixé dans la plaque intermédiaire (10).

7. Appareil suivant la revendication 1, caractérisé en ce que le boîtier (2) comporte, entre le premier (1) et le deuxième éléments (4) capteurs, un canal (8) pour du carburant ayant une partie en oblique qui imprime au mélange qui passe un mouvement de rotation.

8. Appareil suivant la revendication 1, caractérisé en ce que le boîtier (2) et la plaque intermédiaire (10) sont en aluminium et sont revêtus de cuivre et de nickel.

9. Appareil suivant la revendication 1, caractérisé en ce que l'élément (1,4) capteur est en un alliage de fer et de nickel.
